# EUROPEAN PATENT APPLICATION

(11) **EP 4 389 039 A1**
(43) Date of publication of application: **26.06.2024**
(21) Application number: 23216739.5
(22) Date of filing: 14.12.2023
(51) Int. Cl.: A61B 18/12, H03M 11/24, A61B 18/14, A61B 18/00

(54) **DEVICE FOR DETECTING ACTIVATION OF AN INSTRUMENT SWITCHING MEANS OF AN ELECTROSURGICAL INSTRUMENT, ELECTROSURGICAL GENERATOR, DETECTING METHOD AND OPERATING METHOD**

(30) Priority: 23.12.2022 US 202263434992 P
(71) Applicant: Olympus Winter & Ibe GmbH, 22045 Hamburg (DE)
(72) Inventor: Ramin, Daniel, 14558 Nuthetal (DE); Faehsing, Thomas, 12305 Berlin (DE)
(74) Representative: Eisenführ Speiser

(57) **Abstract**

The present invention relates to a device (100) for detecting activation of an instrument switching means (SW1, SW2) of an electrosurgical instrument (20), comprising: a power converter (110) for generating an electrical measurement current (I_{M}), wherein the power converter (110) being configured with at least a first and a second controllable switching means (M1, M2, M3, M4);a control device (120) for actuating the controllable switching means (M1, M2, M3, M4) of the power converter (110), at least first and second instrument switching means (SW1, SW2), wherein the first and second instrument switching means (SW1, SW2) being electrically connected to the power converter (110), and an evaluation device (130) for evaluating the generated measurement current, **characterized in that** the power converter (110) and the instrument switching means (SW1, SW2) are connected in the way that a first measurement current (I_{M1}) establishes along a first current path (140) through the first controllable switching means (M1) and through the first instrument switching means (SW1) when the first controllable switching means (M1) and the first instrument switching means (SW1) are electrically conductive, and no measurement current (I_{M}) establishes when the first controllable switching means (M1) and the second instrument switching means (SW2) are electrically conductive, and a second measurement current (I_{M2} ) establishes along a second current path (150) through the second controllable switching means (M2) and through the second instrument switching means (SW2) when the second controllable switching means (M2) and the second instrument switching means (SW2) are electrically conductive, and no measurement current (I_{M} ) establishes when the second controllable switching means (M2) and the first instrument switching means (SW1) are electrically conductive, and wherein the evaluation device (130) is adapted to detect whether the generated measurement current (I_{M} , I_{M1} , I_{M2} ) is established along the first current path (140) or along the second current path (150).

## Description

The invention relates to a device for detecting activation of an instrument switching means of an electrosurgical instrument. The invention also relates to an electrosurgical generator comprising a device for detecting activation of an instrument switching means of an electrosurgical instrument. The invention also relates to a method for detecting activation of an instrument switching means of an electrosurgical instrument. The invention also relates to a method for operating a device for detecting activation of an instrument switching means of an electrosurgical instrument.

Electrosurgical generators for operating electrosurgical instruments are generally known. Such generators are used as power supply for handheld electrosurgical instruments connected the generator. Such generators are also used for monitoring the correct operation of the connected electrosurgical instrument. The electrosurgical generators are also adapted to monitor user interactions at the generator itself or they are adapted to monitor user interactions at the electrosurgical instrument or at other user interaction means coupled the generator, such as footswitches.

It is for example known that the electrosurgical instruments include instrument switching means, such as pushbuttons or hand switches. Those switching means are used for switching between a cutting mode and a coagulation mode for instance. In the cutting mode, the electrosurgical generator supplies the electrosurgical instrument with a specific high frequency current for cutting tissue. In the coagulation mode, the electrosurgical generator supplies the electrosurgical instrument with a specific high frequency current for coagulating tissue. Therefore, electrosurgical instruments, which are connected to an electrosurgical generator, often have pushbuttons or hand switches for activating different operational modes of the instrument such as cutting or coagulation.

It is known that the instrument switching means are connected to the generator by individual (connection) lines with the generator, for example one line per switching means. Alternatively, the instrument switching means are connected to the generator by a common (connection) line over an instrument connector with the generator. In the second variant, when a common line is used, it is necessary to implement circuitry for detecting, which of the switching means was activated by the user. For that purpose, it is known to implement a diode prior to each pushbutton or hand switch of the instrument in forward direction or in reverse direction. The forward direction is also known as forward bias. The reverse direction is also known as reverse bias. The common line can also be understood as a (electrical) common connection line.

In order to distinguish the switching means of the instrument and in order to monitor, if the switching means of the instrument have been activated or not, an alternating voltage is applied to the common line in known solutions. For example, it is known, to monitor a square wave signal applied to the common line with a frequency of approximately 200 Hertz. This signal has a positive or a negative polarity to the ground as reference point. Due to changing polarity of the applied signal and due to the specific conducting direction of the diode prior to each switching means, an assignable current flow to the switching means establishes, when the respective switching means of the instrument is pressed by the user.

For example, when two different switching means are part of the instrument, and each switching means includes a diode with a different conducting direction, a first current flow establishes during the time, when the applied alternating signal has a positive polarity and when the respective switching means with the forward biased diode is activated by the user. A current flow through the switching means with the forward biased diode does not establish in the time when the applied alternating signal has negative polarity due to the blocking effect of the diode. Vice versa, a second current flow establishes during the time, when the applied alternating signal has a negative polarity and when the respective switching means with the reverse biased diode is activated by the user. A current flow through the switching means with the reversed biased diode does not establish in the time when the applied alternating signal has positive polarity due to the blocking effect of the diode.

Thus, if a current is detected in the time when the applied alternating signal has positive polarity, it can be concluded that the switching means with the forward biased diode is activated by the user. If a current is detected in the time when the applied signal has negative polarity, it can be concluded that the switching means with the reversed biased diode is activated by the user. This makes it possible to query two manual switching means independently of each other, although they are connect via one common line.

However, the generation and provision of negative voltage for this principle requires increased circuit complexity. In addition, negative currents or voltages must be evaluated, which also increases the circuitry effort. In addition, the evaluation is partially performed by means of analog optocouplers, which must be precisely adjusted and calibrated.

In summary, negative voltages to be generated are disadvantageous for detecting activation of an instrument switching means of an electrosurgical instrument. It is thus desirable to avoid such a negative voltage or current supply in the detection process for detecting an activation of a switching means of an electrosurgical instrument.

Therefore, it is an object of the present invention to provide a solution, which decreases the complexity of the electrical circuitry used for monitoring the state of instrument switching means. In particular, it is an object of the present invention to provide a solution, which does not use negative voltages or currents in the monitoring process of an instrument switching means.

According to a first aspect of the invention a device for detecting activation of an instrument switching means of an electrosurgical instrument according to claim 1 is suggested.

The device can also be understood as monitoring unit or detection system. The device is used to detect, if a switching means of an electrosurgical instrument coupled to an electrosurgical generator is activated or not. An instrument switching means is for example a pushbutton or a hand switch arranged at the electrosurgical instrument. With the instrument switching means, a user is able to select different operational modes of the instrument for instance. The devices is thus configured to detect which specific switch or button was pressed by a user. It is understood that the device is coupled to the electrosurgical generator via a connection line, for example via a common connection line as described above.

The electrosurgical generator is used for driving an electrosurgical instrument. The electrosurgical generator supplies said instrument with electrical power, for instance, from an electrical supply grid or from an electrical storage device, such as a battery.

The device comprises a power converter for generating an electrical measurement current, wherein the power converter being configured with at least a first and a second controllable switching means. A measurement current can synonymously also be denoted as measure current. A power converter is generally understood as an electrical or electro-mechanical device for converting electrical energy by means of controllable switches. For example, DC-to-DC, DC-to-AC, AC-to-DC and AC-to-AC are known power converters. The suggested power converter includes at least two controllable switching means, which can be also understood as control switches. The power converter is for example a DC-to-AC converter with a H-bridge topology or the like. The controllable switching means are for example MOSFETs (metal-oxide-semiconductor field-effect transistor), IGBTs (insulated-gate bipolar transistor), BJTs (Bipolar junction transistor) or other electronic switches. The current that is generated by means of the power converter can also be understood as a test current, testing current, measured current or measuring current. Based on the generated electrical measurement current it can be determined, if at least a first or second instrument switching means was activated by the user. The power converter is preferably arranged in an electrosurgical generator. It is also understood that the power converter is supplied by a voltage or current source. The voltage or current source is for example a rectified DC-voltage. The power converter is preferably operated with a voltage at or below 12 volts.

The device further comprises a control device for actuating the controllable switching means of the power converter. The control device can also be understood as control unit, gate driver, driver circuit or trigger circuit or the like. The control device is adapted to provide control signals for actuating the controllable switching means of the power converter. The control device is configured to control each of the switching means of the power converter individually by means of a control signal. It is also possible that the control device provides one or more common control signals to one or more of the switching means of the power converter. The control device actuates the controllable switches of the power converter in a clocked manner. For example, the first controllable switching means is activated by the control device in a first switching phase and the second controllable switching means is activated in a second switching phase at a different time. The term switching phase can also be understood as clock cycle. It is understood that short circuits shall be avoided during the clocked control of the controllable switching means in normal operation. However, a short-circuit operation may be desired in order to determine a noise current.

The device further comprises at least first and second instrument switching means, wherein the first and second instrument switching means being electrically connected to the power converter. It is thus suggested that the electrosurgical instrument includes at least two instrument switching means, for example two different hand switches arranged at a handle of the electrosurgical instrument. As said before, with the two different instrument switching means for instance different operating modes may be selected by the user or operator of the instrument. Preferably, the switching means of the instrument are electrically coupled with the power converter by means of a common connection line.

The device further comprises an evaluation device for evaluating the generated measurement current. The evaluation device can also be understood as measuring unit or measuring system. The evaluation device is configured to measure or to monitor the current or voltage that is generated with the power converter.

An essential aspect of the invention is how the previously described components are electrically interconnected.

It is proposed that the power converter and the instrument switching means are connected in the way that a first measurement current establishes along a first current path through the first controllable switching means and through the first instrument switching means when the first controllable switching means and the first instrument switching means are electrically conductive, and no measurement current establishes when the first controllable switching means and the second instrument switching means are electrically conductive.

It is thus suggested that the first instrument switching means and the second instrument switching means are wired over a first current path in such a way that although the first controllable switching means of the power converter and the second instrument switching means of the electrosurgical instrument are conductive, no measurement current establishes. One possibility to achieve such a behavior is for example that the first instrument switching means and the second instrument switching means are electrically connected in parallel, and the first instrument switching means has a different conducting direction than the second instrument switching means. Thus, a first measurement current flowing along the first current path only establishes when the first controllable switching means of the power converter and the first instrument switching means of the instrument are electrically conductive. This way, when a measurement current is detected or measured during the time when the first controllable switch of the power converter is conductive, it can be concluded that the first instrument switching means was activated.

Furthermore, it is proposed that the power converter and the instrument switching means are connected in the way that a second measurement current establishes along a second current path through the second controllable switching means and through the second instrument switching means when the second controllable switching means and the second instrument switching means are electrically conductive and no measurement current establishes when the second controllable switching means and the first instrument switching means are electrically conductive.

It is thus suggested that the first instrument switching means and the second instrument switching means are wired over a second current path in such a way that although the second controllable switching means of the power converter and the first instrument switching means of the electrosurgical instrument are conductive, no measurement current establishes. As said before, one possibility to achieve such a behavior is for example that the first instrument switching means and the second instrument switching means are electrically connected in parallel, and the first instrument switching means has a different conducting direction than the second instrument switching means. Thus, a second measurement current flowing along the second current path only establishes when the second controllable switching means of the power converter and the second instrument switching means of the instrument are electrically conductive. This way, when a measurement current is detected or measured during the time when the second controllable switch of the power converter is conductive, it can be concluded that the second instrument switching means was activated.

For distinguishing whether the generated measurement current is established along the first current path, or along the second current path, the evaluation device is used. It is suggested that the evaluation device is adapted to detect whether the generated measurement current is established along the first current path or along the second current path.

One possible way to distinguish whether the generated measurement current established along the first current path or along the second current path is to evaluate the switching phases or clock cycles of the controllable switches of the power converter and the measurement current. If for example a measurement current is detected in the clock cycles when the first controllable switch of the power converter is active, it can be concluded that the first instrument switching means is activated, due to the abovementioned wiring and different conducting directions of the instrument switching means. Vice versa, if a measurement current is only detected in the clock cycles when the second controllable switch of the power converter is active, it can be concluded that the second instrument switching means is activated.

With a wiring topology as previously described, it is possible to detect the activation of the two instrument switching means without using a negative voltage. The suggested device can be implemented with less circuitry complexity and in addition, complex analog circuitry suitable for negative voltages and currents can be avoided. Components suitable for negative voltages can thus be avoided.

Preferably, the first instrument switching means and the second instrument switching means are electrically connected in parallel, and the first instrument switching means has a different conducting direction than the second instrument switching means. The conducting direction is a forward direction or a reverse direction. Both directions are generally known. The forward direction is also known as forward bias. The reverse direction is known as reverse bias. It is thus suggested to connect the two instrument switching means parallel, but with different conducting directions. It is understood that when a voltage with a predetermined direction is applied on the two parallel instrument switching means, a current only flows through one of the instrument switching means.

Preferably, the first instrument switching means is formed with a first actuatable switch and a first diode having a first conducting direction, and the second instrument switching means is formed with a second actuatable switch and with a second diode having a second conducting direction, wherein the first and second conducting directions are different. The first actuatable switch and/or the second actuatable switch is for example a toggle switch, a pushbutton, a hand switch or the like. A diode is generally known. A diode is an electronic component that conducts current primarily in one direction. It has low resistance in one direction, and high resistance in the other. It is thus suggested that the a first diode with a first conducting direction is arranged in series to the first instrument switching means and a second diode with a different conducting direction is arranged in series to the second instrument switching means. For example, the first diode conducts a current in forward direction and the second diode conducts a current in reverse direction.

In addition or alternatively, the first instrument switching means is formed with a first specific resistance or resistor and/or the second instrument switching means is formed with a second specific resistance or resistor. It is also suggested that further instrument switching means are formed in the same way as the first and second switching means, thus with an actuatable switch, a diode and/or a specific resistance or resistor. By means of the specific resistor, a unique current flow per instrument switching means can be provided in order to distinguish the instrument switching means with diodes having the same conducting direction.

Preferably the evaluation device is adapted to evaluate the current amplitude of the measurement current Iₘ. In this way, the current amplitude can be evaluated to distinguish the instrument switching means having the same conducting direction. A different current amplitude can be set with the said resistors.

Preferably, the power converter is formed with an H-bridge with at least four controllable switching means. A H-bridge is generally known and can be synonym be understood as bridge circuit. A H-bridge or bridge circuit is a topology of electrical circuitry in which two circuit branches (usually in parallel with each other) are "bridged" by a third branch connected between the first two branches at some intermediate point along them. A H-bridge is an electronic circuit that switches the polarity of a voltage applied to a load. The load in this case is the first instrument switching means and the second instrument switching means. The H-bridge includes four controllable switching means. With these four controllable switching means in general five different switching states can be set: A forward mode in which the first and fourth switching means are active, a reverse mode in which the second and third switching means are active, an idle mode in which either all switches are inactive or only one of the four switches is active, a bridge short circuit mode in which the second and fourth switching means or the first and third switching means are active, and a short circuit mode in which the first and second switching means and/or the third and fourth switching means are active.

Therefore, in the forward mode, a positive voltage can be applied across the first and second instrument switching means and in the reverse mode a negative voltage can be applied across the first and second instrument switching means. Due to the different conducting direction of the first and second instrument switching means, current only flows across one of the instrument switching means. Vice versa, when the othertwo switches of the H-bridge are closed, a reversed voltage is applied across the first and second instrument switching means. Again, due to the different conducting direction of the first and second instrument switching means, current flows only across one of the switching means.

By using a H-bridge, a positive and negative voltage is applied only across the first and second instrument switching means. The rest of the circuitry works with positive voltages only. This way complex circuitry for negative voltages can be avoided since only the first and second instrument switching means and the diodes receive positive and negative voltage.

Particularly preferred at least the first instrument switching means and the second instrument switching means are electrically conductively connected to a midpoint of the H-bridge. It is thus suggested to connect the first instrument switching means and the second instrument switching means as a load to the H-bridge. The first instrument switching means and the second instrument switching means are thus arranged in the bridge path of the H-bridge. As said before, a H-bridge or bridge circuit is a topology of electrical circuitry in which two circuit branches (usually in parallel with each other) are bridged by a third branch connected between the first two branches at some intermediate point along them. If the aforementioned terminology is used, then the first instrument switching means and the second instrument switching means are arranged in the third branch connected between the two other branches. With this arrangement, a positive and negative voltage can be established across the first instrument switching means and the second instrument switching means by actuating the controllable switches only. A need for a negative voltage source is thus omitted.

Preferably, the control device is configured to actuate the controllable switching means of the power converter clocked, wherein the control device actuates at least the first controllable switching means in a first switching phase and actuates at least the second switching means in a second controllable switching phase. It is thus suggested that the control devices repeatedly actuates the controllable switching means of the power converter and activates alternating at least the first switching means of the power converter in the first switching phase and the second switching means of the power converter in the second switching phase. Switching phase can also be understood as clock cycle or clock phase.

Preferably, the control device is configured to simultaneously actuate the first controllable switching means and a fourth controllable switching means in a first switching phase and to simultaneously actuate the second controllable switching means and a third controllable switching means in a second switching phase. It is thus suggested that the control device toggles the four switching means of the power converter between forward mode and reverse mode. This way in an alternating manner a positive voltage is applied across the first and second instrument switching means having a specific current flow direction. Therefore, it is suggested to alternate between the two modes constantly. Preferably, the control devices actuates the controllable switching means of the power converter repeatedly over time. Thus, it is suggested to apply a continuously switchover over time between the forward mode and reverse mode of the power converter by means of the control device.

Preferably, the control device is adapted to actuate the controllable switching means of the power converter clocked with a clock frequency in a range from 50Hz to 1 kHz, in particular preferred with a clock frequency of 200Hz or 250Hz. Clocking the controllable switching means of the power converter with a clocking frequency in the suggested frequency range provides a good tradeoff between a fast detection of activation of the switches and good electromagnetic compatibility (EMC).

Preferably, the evaluation device is configured to detect the activation of the first instrument switching means and/or to detect the activation of the second instrument switching means based on an evaluation of a present switching phase of the controllable switching means of the power converter and based on the measurement current. If a measurement current repeatedly occurs only in specific switching phase of the power converter, the evaluation device is able to conclude which of the instrument switching means was activated. For example, when the first and fourth controllable switching means are active, thus the forward mode is set, and a measurement current is detected, the evaluation device concludes that the instrument switching means with the forward bias diode is activated. If a measurement current only flows in reverse mode, the instrument switching means with the reversed bias diode is activated. The threshold values can be stored in the device or programmed into it, for example in the evaluation device.

In addition or alternative it is preferred that the evaluation device is adapted to calculate a difference between the measured measurement current in a first switching phase and a measured measurement current in a second switching phase, and if the calculated difference exceeds a pre-defined threshold an activation of the first or second instrument switching means is determined with the evaluation device. It is understood that the threshold or threshold value for the current can be stored in the device or programmed into it, for example in the evaluation device. The first switching phase is for example a switching phase in which the power converter is set in forward mode and the second switching phase is for example a switching phase in which the power converter is set in reverse mode, or vice versa. Thus, if the calculated difference exceeds the pre-defined threshold, either the first instrument switching means is pressed or the second instrument switching means is pressed.

According to one aspect the evaluation device is adapted to calculate a reference current in a reference switching phase forming a bypass of the electrosurgical instrument, and wherein in particular the evaluation device is adapted to calculate a difference between the measured measurement current in the first switching phase and the reference current and/or a difference between the measured measurement current in the second switching phase and the reference current, to thereby reduce or eliminate high frequency noise.

It was found that noise can occur. By calculating the difference to the reference current, having similar noise, the noise or a higher level due to noise, will be reduced making the wanted signal to stick out more clearly.

Preferably, a threshold value is implemented for the first measurement current and/or the second measurement current, and wherein the evaluation device evaluates the first instrument switching means and/or the second instrument switching means as activated when the threshold value is reached. In order to assure a correct and error free detection of the activation of the instrument switching means measurement current threshold values are defined. If the measurement current reaches or exceeds the threshold value an activation of an instrument switching unit is assumed. This way parasitic currents or electromagnetic couplings into the circuitry can be suppressed.

Preferably, at least one further instrument switching means is connected in parallel to the first and second instrument switching means, and the further instrument switching means has a different current passing direction than the second instrument switching means and/or first instrument switching means. It is thus suggested that the instrument switching means include more than two instrument switching means. The further switching means are connected parallel to the first and second instrument and they are constructed analogously as described before to the first and second instrument switching means. Thus, a cascaded circuit is proposed.

In addition or alternatively any diode each limiting the current through the first and the second instrument switching means to a particular direction, could either be located close to the first and the second instrument switching in the electrosurgical instrument, or it can be located in the electrosurgical generator to which the electrosurgical instrument is connected.

According to a first further aspect of the invention, an electrosurgical generator is suggested comprising a device for detecting activation of an instrument switching means of an electrosurgical instrument, wherein the device is formed according to one of the preceding embodiments. As to the advantages, preferred embodiments and details of this further aspect and its preferred embodiments, reference is made to the corresponding advantages, preferred embodiments and details described above. The electrosurgical generator is used as power supply for electrosurgical instruments connected the generator. The electrosurgical generator comprises an instrument terminal for connecting and electrically supplying the electrosurgical instrument. The instrument terminal can be understood as a connector or port. In operation, the instrument is connected to the terminal in order to provide an electrical connection between the instrument and the generator. The generator is configured to supply the instrument with current or voltage electrically. The generator is connected for that purpose for example with an electrical supply grid and includes a power converter for power supply of the instrument.

According to a second further aspect of the invention, a method for detecting activation of an instrument switching means of an electrosurgical instrument is suggested, wherein the instrument switching means comprises at least a first and second instrument switching means, wherein the first and second instrument switching means being electrically connected to a power converter, and first measurement current establishes along a first current path through the first controllable switching means and through the first instrument switching means when the first controllable switching means and the first instrument switching means are electrically conductive, and no measurement current establishes when the first controllable switching means and the second instrument switching means are electrically conductive, and a second measurement current establishes along a second current path through the second controllable switching means and through the second instrument switching means when the second controllable switching means and the second instrument switching means are electrically conductive, and no measurement current establishes when the second controllable switching means and the first instrument switching means are electrically conductive.

The detection method comprises the first step: Generating an electrical measurement current by means of the power converter, wherein the power converter being configured with at least a first and a second controllable switching means.

The detection method comprises the further step: Actuating the controllable switching means of the power converter by means of a control device.

The detection method comprises the further step: Detecting whether the generated measurement current is established along the first current path or along the second current path by means of an evaluation device for evaluating the generated measurement current.

As to the advantages, preferred embodiments and details of this further aspect and its preferred embodiments, reference is made to the corresponding advantages, preferred embodiments and details described above.

Preferably, the detecting step comprises the step: Detecting the activation of the first instrument switching means and/or to detect the activation of the second instrument switching means based on an evaluation of a present switching phase of the controllable switching means of the power converter and based on the measurement current.

According to a third further aspect of the invention, a method for operating a device for detecting activation of an instrument switching means of an electrosurgical instrument is suggested, wherein the device is formed according to one of the preceding embodiments. In a first step a measuring of an idle current as measurement current is performed, when all controllable switching means are set into a non-conductive state. This step is performed to exclude a faulty circuit. The idle current shall not exceed a pre-defined threshold for the idle current, otherwise the circuit has a failure.

In a second step a measuring of a noise current as measurement current is performed, when all controllable switching means are set into a conductive state. In this step, all controllable switching means of the power converter and thus all paths of the power converter are pulled to ground. This way a noise current can be determined. If the noise current is determined this current can be filtered for better measuring results.

In a further step a repeated switchover between a forward mode and a reverse mode of the power converter is performed, wherein in the forward mode at least the first controllable switching means of the power converter is conductive and optional a or the fourth controllable switching means is conductive, and wherein in the reverse mode at least the second controllable switching means of the power converter is conductive and optional a or the third controllable switching means is conductive. As to the advantages, preferred embodiments and details of this further aspect and its preferred embodiments, reference is made to the corresponding advantages, preferred embodiments and details described above.

Preferred embodiments shall now be described with reference to the attached figures, in which
- Fig. 1: shows an electrosurgical generator and an electrosurgical instrument connected to the electrosurgical generator according to an embodiment.
- Fig. 2: shows a schematic circuit diagram of the device for detecting activation of an instrument switching means of an electrosurgical instrument according to the invention.
- Fig. 3: shows a diagram in which control signals are illustrated in principle and a diagram in which current measurements are illustrated in principle, when no instrument switching means is activated.
- Fig. 4: shows a schematic circuit diagram and a first current path, when a first instrument switching means SW1 is activated.
- Fig. 5: shows a diagram in which control signals are illustrated in principle and a diagram in which current measurements are illustrated in principle, when a first instrument switching means SW1 is activated.
- Fig. 6: shows a schematic circuit diagram and a second current path, when a second instrument switching means SW2 is activated.
- Fig. 7: shows a diagram in which control signals are illustrated in principle and a diagram in which current measurements are illustrated in principle, when a second instrument switching means SW2 is activated.
- Fig. 8: shows a schematic circuit diagram and a further current path, when a further instrument switching means SW3 is activated.

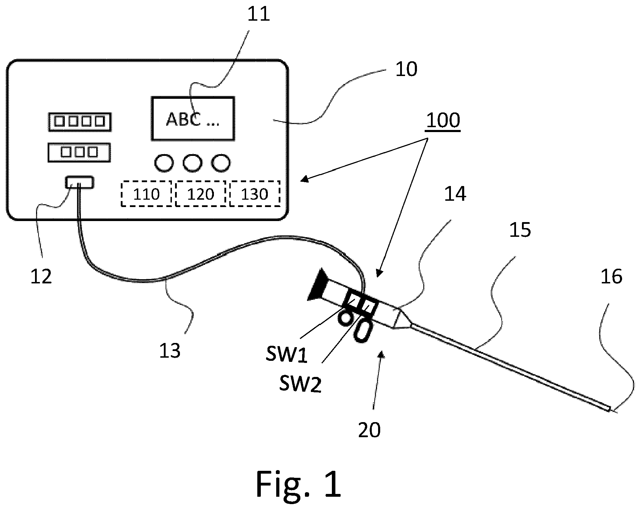

Fig. 1 shows an electrosurgical generator 10 and an electrosurgical instrument 20 connected to the electrosurgical generator 10. The electrosurgical instrument 20 is connected with the electrosurgical generator 10 at the instrument terminal 12 by means of a connection line 13. The connection line 13 may include supply lines and a data transfer line, which are not shown in Fig. 1. The connection line is a common line for connecting the instrument 20 with the generator 10. The generator may also include several instrument terminals, for example, a neutral instrument terminal, a unipolar instrument terminal and a bipolar instrument terminal or the like. The electrosurgical instrument 20 includes a shaft 15 having an active electrode 16 at the end of the shaft 15. The shaft 15 is attached to a handle 14 of the electrosurgical instrument 20. The generator 10 includes a display 11 for displaying text and/or symbols and/or graphics, for example to display optical warnings or operating parameters. The generator 10 also includes an operating device, which is shown as three buttons below the display 11. The operating device may also be a touch display combined with the display 11 or the like. The electrosurgical generator 10 is used for operating the electrosurgical instrument 20.

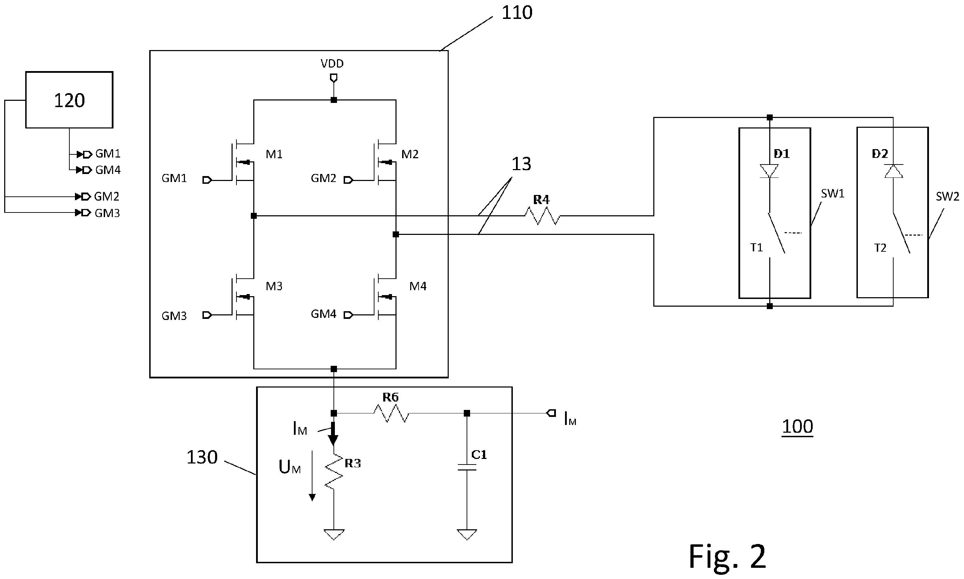

Figure 1 also illustrates the arrangement of the parts of the device for detecting activation of an instrument switching means of an electrosurgical instrument according to the invention. As can be seen in figure 1, the device 100 includes a power converter 110, a control device 120 and an evaluation device 130, which are described hereinafter in more detail, for example in following Figures 2 to 8. The instrument 20 includes a first instrument switching means SW1 and a second instrument switching means SW2. By pressing or activating the instrument switching means SW1 or SW2 a cutting mode or a coagulation mode of the instrument 20 can be set to give an example of the purpose of the instrument switching means.

Fig. 2 shows a schematic circuit diagram of the device 100 for detecting activation of an instrument switching means SW1 or SW2 of an electrosurgical instrument 20.

The device 100 includes power converter 110, a control device 120, an evaluation device 130 and at least a first instrument switching means SW1 and second instrument switching means SW2.

The power converter 110 is used for generating an electrical measurement current I_{M}, wherein the power converter 110 is being configured with at least a first and a second controllable switching means, namely with the first, second, third and fourth controllable switching means M1, M2, M3 and M4.

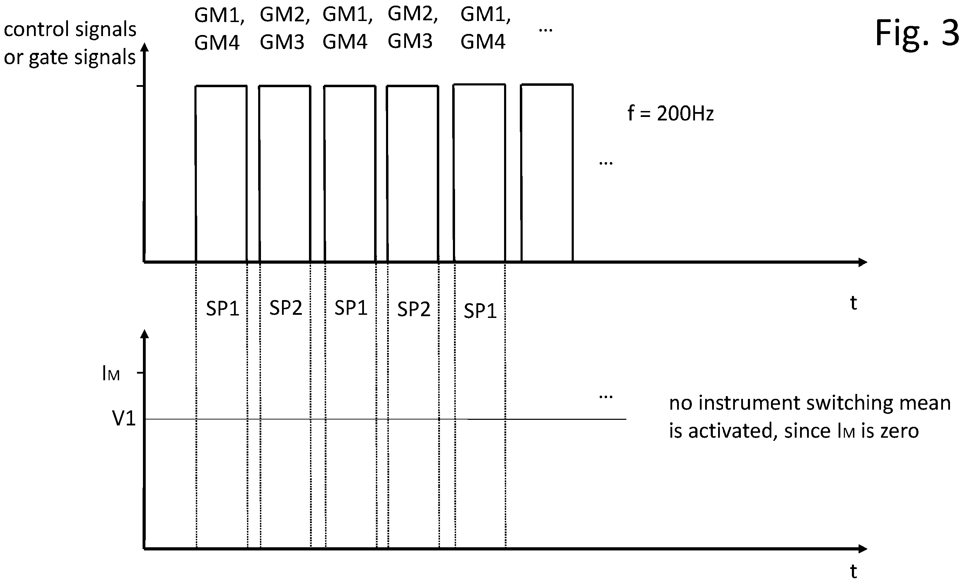

The control device 120 is used for actuating the controllable switching means M1, M2, M3, and M4 of the power converter 110. It can be seen in figure 2 that the control device 120 has four outputs for providing four control signal GM1, GM2, GM3 and GM4 to the controllable switches M1 to M4. The control device 120 is configured to actuate the controllable switching means M1, M2, M3, M4 of the power converter 120 clocked, wherein the control device 120 actuates at least the first switching means M1 in a first switching phase SP1 and actuates at least the second switching means M2 in a second switching phase SP1, as shown for example in Figs. 3, 5 or 7.

The control device 120 is configured to simultaneously actuate the first switching means M1 and a fourth switching means M4 in a first switching phase SP1 (forward mode) and to simultaneously actuate the second switching means M2 and a third switching means M3 in a second switching phase SP2 (reverse mode).

The control device 120 is arranged to actuate the controllable switching means M1, M2, M3, M4 of the power converter 110c locked with a clock frequency f in a range from 50Hz to 1 kHz, in particular with a clock frequency f of 200Hz as shown in Fig. 3, 5 or 7.

The control principle and the clocked control of the control device 120 is shown in Fig. 3, 5 and 7 in more detail.

The device 100 also includes at least first and second instrument switching means SW1 and SW2, wherein the first and second instrument switching means SW1 and SW2 being electrically connected to the power converter 110. The first and second instrument switching means SW1 and SW2 are connected for example over a common connection line 13, as showed in Fig. 1. The resistor R4 illustrates a line resistance of the connection line 13.

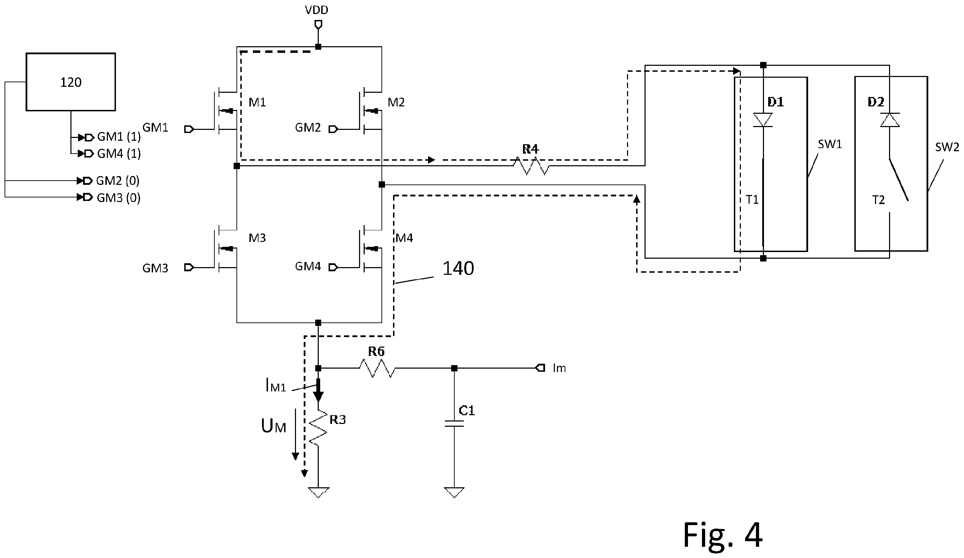

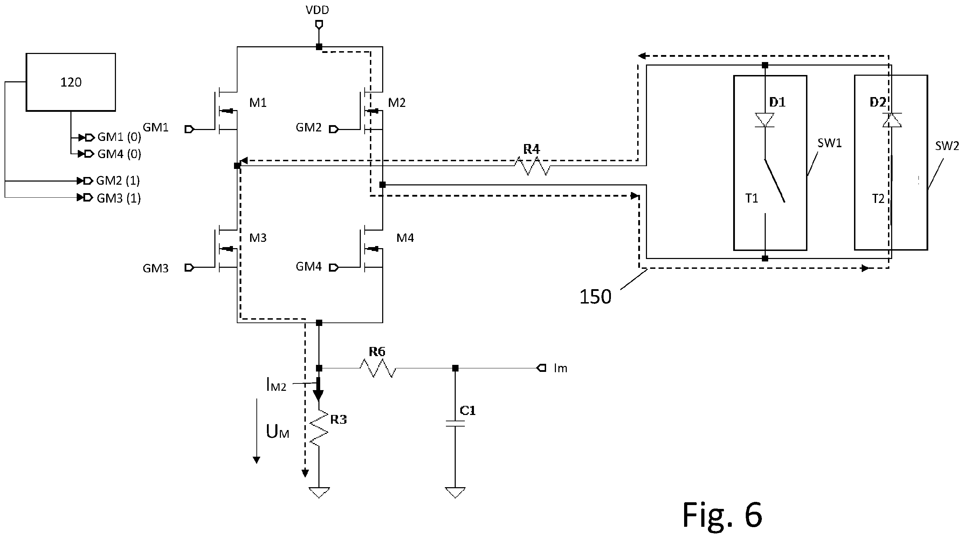

The device 100 also includes an evaluation device 130 used for evaluating the generated measurement current I_{M}, which can be understood as a measuring unit for measuring the measurement current I_{M}. The evaluation device 130 includes optional a signal filter circuit formed with a resistor R6 and a capacity C1. The evaluation device 130 is adapted to detect whether the generated measurement current I_{M}, I_{M1}, I_{M2} is established along a first current path 140 or along the second current path 150. The first current path 140 is shown in figure 4 in more detail. The second current path 150 is shown in figure 6 in more detail.

The evaluation device 130 is configured to detect the activation of the first instrument switching means SW1 and/or to detect the activation of the second instrument switching means SW2 on the basis of an evaluation of a present switching phase SP1 or SP2 of the controllable switching means M1, M2, M3, M4 of the power converter 110 and on the basis of the measurement current I_{M}. This principle is illustrated in Figs. 3, 5 and 7 for instance.

The evaluation device 130 is adapted to calculate a difference between the measured measurement current I_{M1} in a first switching phase SP1 and a measured measurement current I_{M2} in a second switching phase SP2, and if the calculated difference exceeds a pre-defined threshold V1 an activation of the first or second instrument switching means SW1 or SW2 is determined with the evaluation device 130. This principle is illustrated in Figs. 3, 5 and 7 for instance.

The power converter 110 and the instrument switching means SW1, SW2 are connected in the way that a first measurement current I_{M1} establishes along a first current path 140 through the first controllable switching means M1 and the fourth controllable switching means M4 and through the first instrument switching means SW1 when the first controllable switching means M1, the fourth controllable switching means M4 and the first instrument switching means SW1 are electrically conductive. In the showed embodiment, a diode D1 is arranged in forward direction in series to switch button or push button T1, which is part of the instrument switching means SW1.

No measurement current I_{M} establishes when the first controllable switching means M1, the fourth controllable switching means M4 and the second instrument switching means SW2 are electrically conductive, since in the showed embodiment a diode D2 is arranged in series to switch button or push button T2 that blocks a current in the electrical path of the second instrument switching means SW2, even if T2 is activated and conductive.

The power converter 110 and the instrument switching means SW1, SW2 are also connected in the way that a second measurement current I_{M2} establishes along a second current path 150 through the second controllable switching means M2, the third controllable switching means M3 and through the second instrument switching means SW2 when the second controllable switching means M2, the third controllable switching means M3 and the second instrument switching means SW2 are electrically conductive.

No measurement current I_{M} establishes when the second controllable switching means M2, the third controllable switching means M3 and the first instrument switching means SW1 are electrically conductive, since in the showed embodiment a diode D1 is arranged in series to switch button or push button T1 that blocks a current in the electrical path of the first instrument switching means SW1, even if T1 is activated and conductive.

The first instrument switching means SW1 and the second instrument switching means SW2 are electrically connected in parallel, and the first instrument switching means SW1 has a different conducting direction than the second instrument switching means SW2. In figure 2, the first instrument switching means SW1 includes a diode D1 and a push button or hand switch T1 and the second instrument switching means SW1 includes a diode D2 and a push button or hand switch T2. Thus, the first instrument switching means SW1 is formed with a first actuatable switch T1 and a first diode D1 having a first conducting direction and the second instrument switching means SW2 is formed with a second actuatable switch T2 and with a second diode D2, having a second conducting direction, wherein the first and second conducting directions are different.

The power converter 110 in figure 2 is formed with an H-bridge with four controllable switching means M1, M2, M3, M4, and at least the first instrument switching means M1 and the second instrument switching means M2 are electrically conductively connected to a midpoint of the H-bridge.

Fig. 3 shows a diagram in which control signals are illustrated in principle and a diagram in which current measurements are illustrated in principle, when none instrument switching means is activated.

In the upper diagram of figure 3 a plurality of control signals or gate signals GM1 to GM4 are illustrated, which are provided to the controllable switches M1 to M4. The signals GM1 to GM4 are generated with the control device 120. The rectangular shape and course of the signals are only expressing that the controllable switching means M1 - M4 are provided with a high level signal in order to turn the switches on. The control signals GM1 to GM4 can also be formed as digital signal and a high level can be understood as logical "1" for instance. The upper diagram thus illustrates that in a first switching phase SP1 the switches M1 and M4 are provided with a high level signal GM1 and GM4. In the switching phase SP1, the first and fourth controllable switching means M1 and M4 are thus conductive. The second and third controllable switching means M2 and M3 are nonconductive in the switching phase SP1. Although only a single signal for GM1 and GM4 or GM2 and GM3 is illustrated, it is understood the signals GM1 and GM4 can be provided to M1 and M4 or M2 and M3 individually with four signals or with two common signals, depending on the control design. The upper diagram in figure 3 thus shows that a control signal GM1, GM4 is generated in a first switching phase SP1. Afterwards a control signal GM2, GM3 is generated in a second switching phase SP2, and so on. Thus, in the switching phase SP1 the following control pattern is provided to the controllable switches: M1=1, M2=0, M3=0, M4=1. In the switching phase SP2 the control pattern is M1=0, M2=1, M3=1, M4=0. Thus, in the switching phase SP1 the power converter 110 is set in forward mode. In the switching phase SP2, the power inverter 110 is set in reverse mode. This pattern is repeated over time. It is thus suggested that the control device 120 clockwise switches the power converter 110 between forward mode and reverse mode. This control pattern is continuously and alternating provided to the controllable switching means over time. The x-axis in figure 3 is a time axis indicated with the letter "t". The control signals or gate signals GM1 to GM4 are repeatedly generated over time for example with a frequency of 200 Hz.

In the lower diagram of figure 3, the measured measurement current I_{M} over time t is illustrated. A threshold value V1 is implemented for the measurement current I_{M}, and the evaluation device 130 evaluates if the first instrument switching means SW1 or the second instrument switching means SW1 is activated, by comparing the measured measured measurement current I_{M} with the implemented threshold value V1. As can be seen in the lower diagram of figure 3, no measurement current I_{M} is detected, thus, both instrument switching means SW1 and SW2 are not conducting (open), for example as shown in figure 2.

Fig. 4 shows a schematic circuit diagram and a first current path 140, when a first instrument switching means SW1 is activated. It can be seen in figure 4 that the voltage or current source VDD is pulled against ground along the dashed current path 140 over M1 (first controllable switching means), R4 (line resistance), D1 (first diode), T1 (first actuatable switch), M4 (fourth controllable switching means) and R3 (measuring resistor). Figure 4 also shows that the control device 120 sets M1 and M4 with a high level control signal GM1 and GM4 and sets M2 and M3 with a low level control signal GM2 and GM3, indicated with "1" and "0" in brackets. A measurement current I_{M1}, indicated with a black arrow above the resistor R3, establishes along the first current path 140.

For measuring a reference current, M3 and M4 can be pressed simultaneously. In addition with either switch M1 or switch M2 being pressed, the electrosurgical instrument, in particular both switches SW1 and SW2 are bypassed leading to a reference current. Calculating a difference between a current measured in first current path 140 and such reference current leads to a signal with reduced noise level.

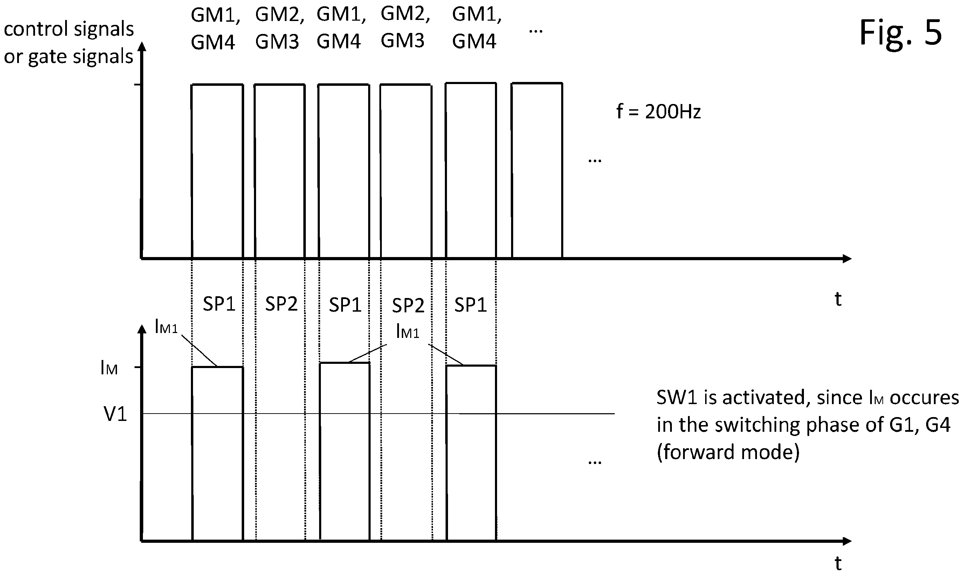

Fig. 5 shows a diagram in which control signals are illustrated in principle and a diagram in which current measurements are illustrated in principle, when a first instrument switching means SW1 is activated. Figure 5 illustrates the control principle of fig. 4. As described in regard to figure 3, in figure 4 the switches M1 to M4 are repeatedly provided with the control signals GM1 to GM4 overtime. Since the first instrument switching device SW1 is pressed, a first measurement current I_{M1} only occurs in the switching phase or switching phases SP1 due to the different conducting directions of the instrument switching means SW1 and SW2. The current I_{M1} reaches the threshold value V1 and thus, the evaluation device detects in this way an activation of the first instrument switching means SW1.

Fig. 6 shows a schematic circuit diagram and a second current path 150, when a second instrument switching means SW2 is activated. It can be seen in figure 6 that the voltage or current source VDD is pulled against ground along the dashed current path 150 over M2 (second controllable switching means), T2 (second actuatable switch), D2 (second diode), R4 (line resistance), M3 (third controllable switching means) and R3 (measuring resistor). Figure 6 also shows that the control device 120 sets M2 and M3 with a high level control signal GM1 and GM3 and sets M1 and M4 with a low level control signal GM2 and GM3, indicated with "1" and "0" in brackets. A measurement current I_{M2}, indicated with a black arrow above the resistor R3, establishes along the second current path 150.

For measuring a reference current, M3 and M4 can be pressed simultaneously. In addition with either switch M1 or switch M2 being pressed, the electrosurgical instrument, in particular both switches SW1 and SW2 are bypassed leading to a reference current. Calculating a difference between a current measured in second current path 150 and such reference current leads to a signal with reduced noise level.

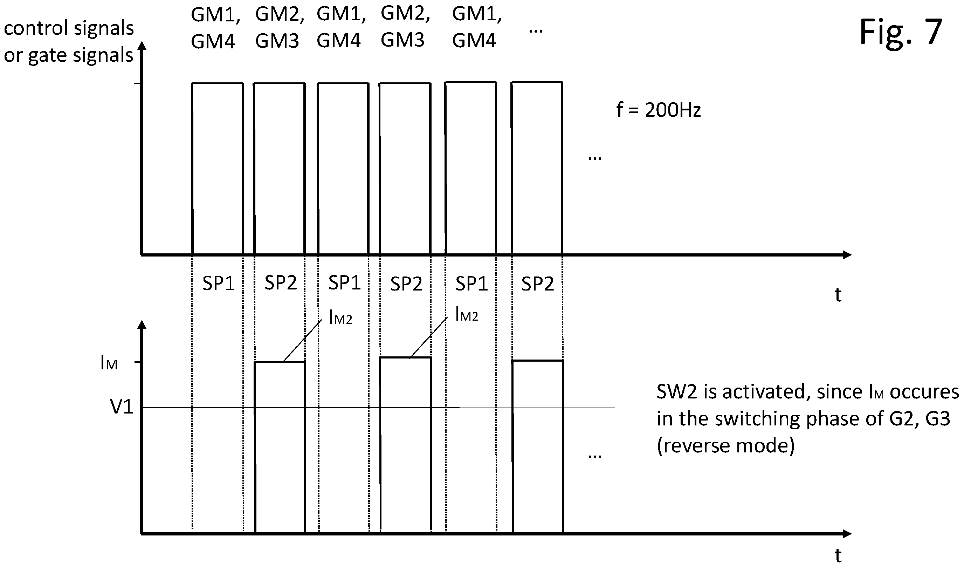

Fig. 7 shows a diagram in which control signals are illustrated in principle and a diagram in which current measurements are illustrated in principle, when a second instrument switching means SW2 is activated. Figure 7 illustrates the control principle of fig. 6. As described in regard to figure 3, in figure 7 the switches M1 to M4 are repeatedly provided with the control signals GM1 to GM4 overtime. Since the second instrument switching device SW2 is pressed, a second measurement current I_{M2} only occurs in the switching phase or switching phases SP2 due to the different conducting directions of the instrument switching means SW1 and SW2. The current I_{M2} reaches the threshold value V1 and thus, the evaluation device detects in this way an activation of the second instrument switching means SW2.

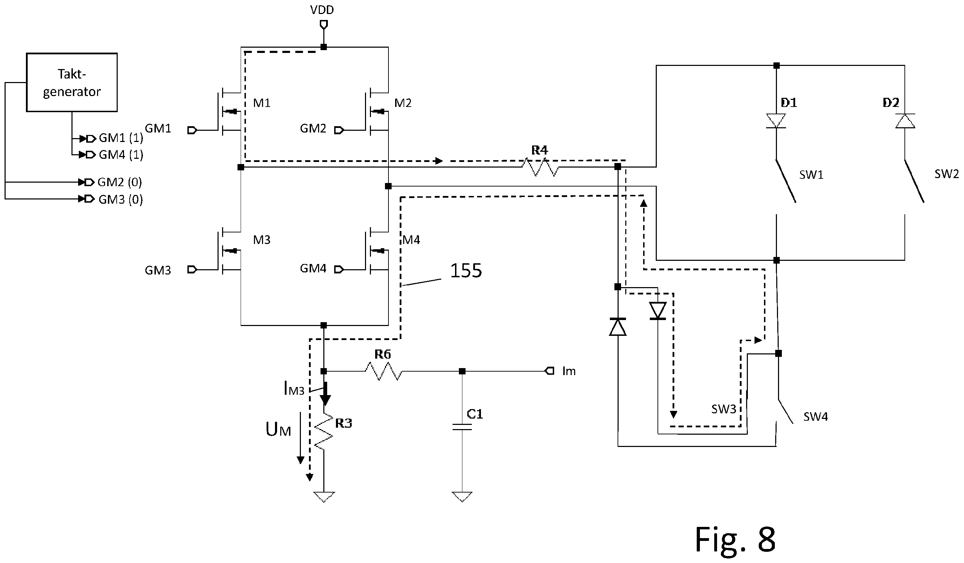

Fig. 8 shows a schematic circuit diagram and a further current path 155, when a further instrument switching means SW3 is activated. Figure 8 shows that at least one further instrument switching means SW3 and/or SW4 is connected in parallel to the first and second instrument switching means SW1, SW2, and the further instrument switching means SW3, SW4 has a different current passing direction than the second instrument switching means SW2 and/or first instrument switching means SW1. In order to distinguish between an activation of SW3 and SW2 or SW4 and SW1 specific resistors may be part of the instrument switching means SW1 to SW4, that are not shown in figure 8. Thus, a specific amount or current amplitude is generated, which can be monitored by the evaluation device. Thus, in general, the evaluation device is adapted to evaluate the current amplitude of the measurement current Iₘ. Thus, it is proposed to distinguish switching means by current amplitude or magnitude.

## Claims

1. Adevice (100) for detecting activation of an instrument switching means (SW1, SW2) of an electrosurgical instrument (20), comprising:
- a power converter (110) for generating an electrical measurement current (I_{M}), wherein the power converter (110) being configured with at least a first and a second controllable switching means (M1, M2, M3, M4);
- a control device (120) for actuating the controllable switching means (M1, M2, M3, M4) of the power converter (110),
- at least first and second instrument switching means (SW1, SW2), wherein the first and second instrument switching means (SW1, SW2) being electrically connected to the power converter (110), and
- an evaluation device (130) for evaluating the generated measurement current (I_{M}),
**characterized in that**
the power converter (110) and the instrument switching means (SW1, SW2) are connected in the way that
(i) a first measurement current (I_{M1}) establishes along a first current path (140) through the first controllable switching means (M1) and through the first instrument switching means (SW1) when the first controllable switching means (M1) and the first instrument switching means (SW1) are electrically conductive, and no measurement current (I_{M}) establishes when the first controllable switching means (M1) and the second instrument switching means (SW2) are electrically conductive, and
(ii) a second measurement current (I_{M2}) establishes along a second current path (150) through the second controllable switching means (M2) and through the second instrument switching means (SW2) when the second controllable switching means (M2) and the second instrument switching means (SW2) are electrically conductive, and no measurement current (I_{M} ) establishes when the second controllable switching means (M2) and the first instrument switching means (SW1) are electrically conductive, and
wherein the evaluation device (130) is adapted to detect whether the generated measurement current (I_{M}, I_{M1}, I_{M2}) is established along the first current path (140) or along the second current path (150).

2. Device (100) according to claim 1, wherein the first instrument switching means (SW1) and the second instrument switching means (SW2) are electrically connected in parallel, and the first instrument switching means (SW1) has a different conducting direction than the second instrument switching means (SW2).

3. Device (100) according to claim 1 or 2, wherein the first instrument switching means (SW1) is formed with a first actuatable switch (T1) and a first diode (D1) having a first conducting direction, and the second instrument switching means (SW2) is formed with a second actuatable switch (T2) and with a second diode (D2) having a second conducting direction, wherein the first and second conducting directions are different, and preferably the first instrument switching means (SW1) is formed with a first specific resistance or resistor (R1) and/or the second instrument switching means (SW2) is formed with a second specific resistance or resistor (R2).

4. Device (100) according to one of the preceding claims, wherein the power converter (110) is formed with an H-bridge with at least four controllable switching means (M1, M2, M3, M4), and preferably at least the first instrument switching means (M1) and the second instrument switching means (M2) are electrically conductively connected to a midpoint of the H-bridge.

5. Device (100) according to one of the preceding claims, wherein the control device (120) is configured to actuate the controllable switching means (M1, M2, M3, M4) of the power converter (120) clocked, wherein the control device (120) actuates at least the first controllable switching means (M1) in a first switching phase (SP1) and actuates at least the second controllable switching means (M2) in a second switching phase (SP2).

6. Device (100) according to one of the preceding claims, wherein the control device (120) is configured to simultaneously actuate the first controllable switching means (M1) and a fourth controllable switching means (M4) in a first switching phase (SP1) and to simultaneously actuate the second controllable switching means (M2) and a third controllable switching means (M3) in a second switching phase (SP2).

7. Device (100) according to one of the preceding claims, wherein the control device (120) is arranged to actuate the controllable switching means (M1, M2, M3, M4) of the power converter (110) clocked with a clock frequency (f) in a range from 50Hz to 1kHz, in particular with a clock frequency of 200Hz or 250Hz.

8. Device (100) according to one of the preceding claims, wherein the evaluation device (130) is configured to detect the activation of the first instrument switching means (SW1) and/or to detect the activation of the second instrument switching means (SW2) on the basis of an evaluation of a present switching phase of the controllable switching means (M1, M2, M3, M4) of the power converter (110) and on the basis of the measurement current (I_{M}); and/or
the evaluation device (130) is adapted to calculate a difference between the measured measurement current in a first switching phase (SP1) and a measured measurement current in a second switching phase (SP2), and if the calculated difference exceeds a pre-defined threshold an activation of the first or second instrument switching means is determined with the evaluation device and/or
the evaluation device (130) is adapted to calculate a reference current in a reference switching phase forming a bypass of the electrosurgical instrument, and wherein in particular
the evaluation device (130) is adapted to calculate a difference between the measured measurement current in the first switching phase (SP1) and the reference current and/or a difference between the measured measurement current in the second switching phase (SP2) and the reference current, to thereby reduce or eliminate high frequency noise.

9. Device (100) according to one of the preceding claims, wherein a threshold value is implemented for the first measurement current and/or the second measurement current, and wherein the evaluation device (130) evaluates the first instrument switching means and/or the second instrument switching means as activated when the threshold value is reached.

10. Device (100) according to one of the preceding claims, wherein at least one further instrument switching means (SW3, SW4) is connected in parallel to the first and second instrument switching means (SW1, SW2), and the further instrument switching means (SW3, SW4) has a different current passing direction than the second instrument switching means (SW2) and/or first instrument switching means (SW1).

11. An electrosurgical generator (10) comprising a device (100) for detecting activation of an instrument switching means (SW1, SW2) of an electrosurgical instrument (20), **characterized in that** the device (100) is formed according to any of the preceding claims 1 to 10.

12. Method for detecting activation of an instrument switching means (SW1, SW2) of an electrosurgical instrument (20), the instrument switching means comprising at least a first and second instrument switching means (SW1, SW2), wherein the first and second instrument switching means (SW1, SW2) being electrically connected to a power converter (110), and
a first measurement current (I_{M1}) establishes along a first current path (140) through the first controllable switching means (M1) and through the first instrument switching means (SW1) when the first controllable switching means (M1) and the first instrument switching means (SW1) are electrically conductive, and no measurement current (I_{M}) establishes when the first controllable switching means (M1) and the second instrument switching means (SW2) are electrically conductive, and
a second measurement current (I_{M2} ) establishes along a second current path (150) through the second controllable switching means (M2) and through the second instrument switching means (SW2) when the second controllable switching means (M2) and the second instrument switching means (SW2) are electrically conductive, and no measurement current (I_{M} ) establishes when the second controllable switching means (M2) and the first instrument switching means (SW1) are electrically conductive, the method comprising the steps of:
- Generating an electrical measurement current (IM) by means of the power converter (110), wherein the power converter (110) being configured with at least a first and a second controllable switching means (M1, M2, M3, M4);
- Actuating the controllable switching means (M1, M2, M3, M4) of the power converter (110) by means of a control device (120);
- Detecting whether the generated measurement current (I_{M}, I_{M1}, I_{M2}) is established along the first current path (140) or along the second current path (150) by means of an evaluation device for evaluating the generated measurement current.

13. Method for detecting activation of an instrument switching means (SW1, SW2) of an electrosurgical instrument (20) according to claim 12, wherein the detecting step comprises:
- Detecting the activation of the first instrument switching means (SW1) and/or to detect the activation of the second instrument switching means (SW2) on the basis of an evaluation of a present switching phase of the controllable switching means (M1, M2, M3, M4) of the power converter (110) and on the basis of the measurement current (I_{M}).

14. Method for operating a device for detecting activation of an instrument switching means (SW1, SW2) of an electrosurgical instrument (20), wherein the device is formed according to one of the preceding claims 1 to 10, wherein the method comprises the steps:
- Measuring an idle current as measurement current (I_{M}), when all controllable switching means are set into a non-conductive state;
- Measuring a noise current as measurement current (I_{M}), when all controllable switching means are set into a conductive state;
- repeated switchover between a forward mode and a reverse mode of the power converter,
wherein in the forward mode at least the first controllable switching means of the power converter is conductive and optional a or the fourth controllable switching means is conductive, and
wherein in the reverse mode at least the second controllable switching means of the power converter is conductive and optional a or the third controllable switching means is conductive.
